Europäisches Patentamt

European Patent Office

Office européen des brevets

(19)

(11) Veröffentlichungsnummer: **0 024 304**
**B1**

(12) **EUROPÄISCHE PATENTSCHRIFT**

(45) Veröffentlichungstag der Patentschrift:
**04.01.84**

(21) Anmeldenummer: **80104167.4**

(22) Anmeldetag: **17.07.80**

(51) Int. Cl.³: **A 61 L 2/12, A 61 L 2/16**

(54) **Vorrichtung zur Sterilisation von Matratzen und grossvolumigen Bett-Textilien.**

(30) Priorität: **18.08.79 DE 2933498**

(43) Veröffentlichungstag der Anmeldung:
**04.03.81 Patentblatt 81/9**

(45) Bekanntmachung des Hinweises auf die Patenterteilung:
**04.01.84 Patentblatt 84/1**

(84) Benannte Vertragsstaaten:
**AT BE CH DE FR GB IT LI NL SE**

(56) Entgegenhaltungen:
**US - A - 3 753 651**

**AM. J. OF HOSPITAL PHARMACY, Nr. 29, August 1972, Seiten 661-672 R.M.G. BOUCHER: "Advances in sterilization techniques"**

(73) Patentinhaber: **Deppe, Hans-Dieter, Dr., Schulstrasse 44, D-4234 Alpen 1 (DE)**

(72) Erfinder: **Deppe, Hans-Dieter, Dr., Schulstrasse 44, D-4234 Alpen 1 (DE)**

(74) Vertreter: **Werner, Hans-Karsten, Dr., Deichmannhaus am Hauptbahnhof, D-5000 Köln 1 (DE)**

Vorrichtung zur Sterilisation von Matratzen und grossvolumigen Bett-Textilien

Die Erfindung betrifft eine neue Vorrichtung zur chemothermischen Desinfektion und Sterilisation von Matratzen und grossvolumigen Bett-Textilien. Die übliche und zunehmende Verwendung von thermolabilen Materialien bei der Herstellung von Matratzen und grossvolumigen Bett-Textilien wie Bettdecken, Kissen etc. verbietet oftmals die Anwendung bekannter Desinfektions- und Sterilisationsverfahren, beispielsweise mit Heissluft, Dampf, grossen Mengen von Aldehydgas, Aldehyddampf oder Äthylenoxydgas. Ausser der Materialunverträglichkeit stört in einigen Fällen auch die Bildung toxischer Rückstände.

Es ist bekannt, dass chemische Desinfektions- und Sterilisationsmittel ihre Wirksamkeit bei Temperaturerhöhung ebenfalls erhöhen und teilweise potenzieren. In der Praxis werden daher Desinfektion und Sterilisation durch mehrstündige, teilweise sogar längere Behandlung mit den Desinfektions- und Sterilisationsmitteln bei erhöhter Temperatur durchgeführt. Diese chemothermischen Desinfektions- und Sterilisationsverfahren ermöglichen zwar das Arbeiten bei niedrigeren Temperaturen als bei thermischen Desinfektions- und Sterilisationsverfahren. Trotzdem sind auch bei kombinierter Anwendung von Chemikalien und Temperatur relativ grosse Mengen Chemikalien und lange Einwirkungszeiten von mehreren Stunden (z. T. Tagen) erforderlich, um einen ausreichenden Effekt zu erzielen. Schliesslich ist auch schon versucht worden, die rein thermische Desinfektion und Sterilisation mit Hilfe von Mikrowellen durchzuführen. Obwohl einzelne Autoren gewisse Effekte beobachtet haben wollen, die über die rein thermische Wirkung der Mikrowellen hinausgehen, so hat die kritische Überprüfung ergeben, dass Mikrowellen für sich allein nur in dem Masse wirksam sind, wie es zur Erwärmung der zu sterilisierenden bzw. desinfizierenden Objekte kommt; vgl. Inhibition and Destruction of the Microbial Cell, W. B. Hugo, Acad. Press London, New York 1971, Seite 302-303.

Aus der US-PS 37 53 651 ist bekannt, versiegelte ärztliche Instrumente anstelle mit Hitze üblicher Herkunft durch Mikrowellen zu sterilisieren. Hierbei wird aber kein besonderer Effekt erzielt gegenüber der herkömmlichen Sterilisation von Kleingeräten. In Amer. J. of Hosp. Pharm. 29, (1972), S. 661-672 wird von einem kombinierten Sterilisationsverfahren unter Benutzung von Ethylenoxid und Mikrowellen gesprochen, wobei als Sterilisationsgüter kontaminierte Lebensmittel erwähnt werden. Diese Literatur wie auch die übrigen besprochenen Veröffentlichungen legen die erfindungsgemässe Vorrichtung nicht nahe.

Besondere Probleme bestehen bei der häufig notwendigen, aber kostspieligen Desinfektion und Sterilisation von Matratzen und grossvolumigen Bett-Textilien in Krankenhäusern, Sanatorien sowie Hotels, die wegen der erheblichen Erschwernisse auch häufig unterlassen wird.

Es bestand somit die Aufgabe, die untenstehend definierte Vorrichtung zu entwickeln, welche möglichst universell einsetzbar ist, die eine Sterilisation mit wenig Chemikalien ermöglicht und darüber hinaus bereits in kurzen Behandlungszeiten eine ausreichende Desinfektion und Sterilisation bewirkt. Diese Aufgabe konnte jetzt überraschenderweise dadurch gelöst werden, dass die erfindungsgemässe Vorrichtung den Einsatz an sich bekannter Desinfektions- und Sterilisationsmittel, vorzugsweise in wässriger oder wässrig alkoholischer Lösung zusammen mit Mikrowellen ermöglicht.

Die Erfindung betrifft somit Vorrichtung zur chemothermischen Sterilisation von Matratzen und grossvolumigen Bett-Textilien mittels Wärme, die durch Mikrowellen erzeugt wird, sowie Zusatz von wässrigen oder wässrig-alkoholischen Lösungen von Desinfektions- und Sterilisationsmitteln, bestehend aus einem vorzugsweise fahrbaren Behälter (1) mit einem verschliessbaren Einlassschlitz (2), einem verschliessbaren Auslassschlitz (3), einer Trommel (4), (4a), einer Einlassklappe (5), einer Auslassklappe (6), einem zentral gelagerten, gegebenenfalls aktiv drehbaren Einsatz (7) mit mindestens einem Mikrowellensender (8), einem Flüssigkeitstank (9) und damit verbundenen Sprühdüsen (10, 10'), ferner aus einer, hinter dem Einlassschlitz (2) angeordneten, angetriebenen Rolle (11) und einer Andruckrolle (12) und gegebenenfalls einer, vor dem Auslassschlitz (3) vorhandenen, weiteren angetriebenen Rolle (11a) und Andruckrolle (12a), wobei die Trommelwand (4) als Rollenwand ausgebildet ist und die innere Trommelwand (4a) über einen Motor (13) angetrieben wird.

Der Einsatz der Vorrichtung unter Kombination dieser beiden Massnahmen führt zu überraschend guten Ergebnissen bezüglich des Verbrauches an Chemikalien sowie extrem kurzer Behandlungszeiten. Weiterhin gestattet die Vorrichtung die Desinfektion und Sterilisation von Objekten, die bisher nur unter grösstem Aufwand desinfiziert und/oder sterilisiert werden konnten, nämlich Matratzen und grossvolumige Bett-Textilien, wenn sie aus Schaumstoff, Gummi oder Kunststoff hergestellt sind. Insbesondere poröse und rissige Materialien können mittels dieser Vorrichtung erstmals materialgerecht, dabei gründlich und obendrein schnell desinfiziert und sterilisiert werden. Besonders gute Erfolgte erzielt man bei intervallmässiger Behandlung durch Mikrowellen, da hierdurch intervallmässig die Desinfektions- und Sterilisationsmittel verdampfen und sich wieder niederschlagen und dadurch besser noch als Gase oder Flüssigkeiten in Risse und Poren eindringen. Der intervallmässige Einsatz der Mikrowellen gestattet weiterhin eine hervorragende Kontrolle der Temperaturen bei den zu behandelnden Objekten, so dass auch thermolabile Materialien erfindungsgemäss desinfiziert und sterilisiert werden können. Beim Einsatz der erfindungsgemässen Vorrichtung werden die zu behandelnden Objekte mit einer Lösung des Desinfektions- und Sterilisationsmittels eingefeuchtet und danach den Mikrowellen ausgesetzt. Da die Mikrowellen auch nicht-metallisches Material durchdringen, werden nicht nur die äusseren, sondern auch die inneren Oberflächen und Geräte gezielt erreicht. Der steile Temperaturgradient bei Mikrowellenerhitzung kleiner Feuchtemengen gestattet es, in vergleichsweise kurzen Zeitspannen von nur wenigen Sekunden die gewünschten Temperaturoptima zu erreichen. Bei intervallmässigem Arbeiten vermögen die Desinfektions- und Sterilisationsmittel vorzugsweise in wässriger oder wässrigalkoholischer Lösung abwechselnd zu verdampfen und zu kondensieren und dabei auch schwer erreichbare Oberflächen im Inneren von porösen Körpern zu erreichen.

Bei der erfindungsgemässen Vorrichtung führt die hohe Wirksamkeit der Kombination von Chemikalien und Mikrowellen zu erheblichen Einsparungen an Desinfektions- und Sterilisationsmitteln sowie Energie. Dies führt nicht nur zu erheblicher Kostensenkung, sondern auch zu einer Minderung der Umweltbelastung.

Die Funktionsweise der Vorrichtung kann in verschiedener Weise beschrieben werden:

1. In einer Kammer wird unterhalb des eingebrachten, zu entkeimenden Materials ein Desinfektionsmittelreservoir mit Hilfe der Mikrowellen erhitzt und zur Verdunstung gebracht. Gegebenenfalls durch Ventilation können diese Nebel umgewälzt werden und sich dabei auf den zu entkeimenden Materialien niederschlagen. Die so benetzten Materialien können jetzt den Mikrowellen ausgesetzt und dabei in optimaler Weise desinfiziert und sterilisiert werden.

2. Die Desinfektions- und Sterilisationsmittel werden durch ein Düsensystem auf oder in die Objekte gesprüht, wobei dieser Sprühvorgang vor und/oder während der Aussendung von Mikrowellen geschehen kann.

3. Das Material kann ausserhalb der Mikrowellenkammer in verschiedenster Weise in Hand oder durch Wascheinrichtungen befeuchtet werden.

4. Die Befeuchtung kann ggf. auch intervallmässig durch Einblasen entsprechender feuchter Dämpfe sowie mit Hilfe von Druck und/oder Temperaturdifferenzen bewerkstelligt werden.

5. Die keimtötende Desinfektionsmittellösung kann in Ampullen aus durchstrahlbarem Material gefüllt in die Kammer eingebracht werden. Durch die Temperaturerhöhung können die Ampullen an Sollbruchstellen platzen und der Inhalt verpuffen. Vorteilhaft sind auch geeignete Druckventilöffnungen einsetzbar, so dass diese nachfüllbaren Ampullen mehrfach hintereinander in gleicher Weise wiederverwendet werden können. Insbesondere bei intervallmässigem Einsatz kann so eine einfache Dosierung von frischem Desinfektions- und Sterilisationsmittel erfolgen.

6. Für die erfindungsgemäss sterilisierbaren Objekte wie Matratzen, Bettzeug, Bettgestelle und Transportbehälter usw. kommen Kammern in Betracht, in welche die zu behandelnden Objekte manuell und/oder automatisch eingebracht und wieder entfernt werden. Insbesondere wenn diese Teile zusätzlich Metall enthalten, kann durch intervallmässige Behandlung eine zu hohe Erhitzung durch Energieabsorption der Metallteile vermieden werden. Die Vorrichtung ist somit sehr flexibel einsetzbar und kann in weiten Grenzen an die erfindungsgemässe Aufgabe angepasst werden.

Bei mehrfacher Anwendung der Mikrowellen wird sich von Stufe zu Stufe das Temperaturniveau der wässrigen Präparatlösungen und der zu behandelnden Objekte erhöhen. Diese Temperatursteigerung kann in sinnvoller Weise dazu verwendet werden, nach der Desinfektion und Sterilisation das Abtrocknen des Materials und das Entfernen feuchter Nebel zu beschleunigen.

Als Desinfektions- und Sterilisationsmittel eignen sich viele bekannte Lösungen von Wirkstoffen. Je nach Zweck und Aufgabenstellung werden sie ausgewählt, um entweder in kurzen Zeitspannen Keimfreiheit oder Keimarmut zu erreichen und/oder über lange Fristen Keimwachstum und Rekontamination zu vermeiden oder einzuschränken. Die meisten der bekannten Desinfektionsmittel schliessen zwischen ihren Eigenschaften und Aufgaben einen anwendungsbegründeten Kompromiss. Durch den steuerbaren Lauf der erfindungsgemässen Vorrichtung, insbesondere bei intervallmässiger Anwendung, ist es möglich, Wirkstoffe mit Langzeit- und/oder Kurzzeitcharakter optimal zu beeinflussen. Das Gerät kann insbesondere so gesteuert werden, dass man stets unterhalb von 100°C, vorzugsweise im Bereich 50 bis 80°C bleibt.

Ausser den bisher bekannten Stoffen ist nun der Einsatz von solchen Substanzen möglich, die erst unter der Einwirkung der Mikrowellen antimikrobiell wirksam werden oder wirksame Komponente freisetzen. Ebenso wird der Einsatz toxisch bedenklicher Wirkstoffe durch die Möglichkeit des Zusatzes von inaktivierenden Reagenzien erheblich erleichtert oder ermöglicht, bzw. durch die Mikrowellenbehandlung in nichttoxische Komponenten gespalten.

Als Sterilisations- bzw. Desinfektionslösungen haben sich insbesondere wässrige und wässrig alkoholische Lösungen und Emulsionen oder Dispersionen bewährt. Ggf. können auch Kombinationen von Wirkstoffen eingesetzt werden sowie gewünschtenfalls Textilpflegemittel, Antikorrosiva und Tenside zugefügt werden. Als zusätzliche Komponenten kommen beispielsweise ausser Wasser und niedere aliphatische Alkohole Diole und ihre Ester, Äther und Ätherester, aromatische Alkohole und ihre Derivate wie Phenylalkohol, Benzylalkohol, Chlorbenzylakohol, phenolische Wirkstoffe wie arylierte und alkylierte Phenole, Biguanide und ihre Derivate, 8-Hydroxichinolin und Derivate sowie quaternäre Ammonium- und Phosphoniumverbindungen in Frage.

Als halogenabspaltende Verbindungen können Hypochlorit, Chloramine, Chlorisocyanursäuren, Jod und Jodophore eingesetzt werden. Konservierungsmittelsäuren, deren Salze und Ester wie Benzoesäure, Salizylsäure, Sorbinsäure, Ameisensäure, aldehydische Wirkstoffe, Formaldehyd. Glyoxal, Glutardialdehyd, Succinaldehyd, ringförmige Verbindungen mit aldehydischen Gruppen und Aldehydgruppen abspaltende Substanzen wie Halbacetale und Acetale können eingesetzt werden. Bevorzugt können auch verwendet werden:
wasserlösliche anorganische Peroxide wie $H_2O_2$, Ammoniumperoxidisulfat, Kaliumperoxodisulfat,

Natriumperoxodisulfat, H$_2$O$_2$-Harnstoffaddukte u.a., oder organische Peroxide mit ausreichender Wasserlöslichkeit und/oder unter Lösungsvermittlung herbeizuführender Wasserlöslichkeit.

Als besonders bevorzugt gelten solche Peroxide, die schon durch Temperaturerhöhung auf 30°C bis 80°C innerhalb von Minuten erhöhte Radikalbildung und Zerfall zeigen. Die toxischen Eigenschaften der entstehenden Reststoffe bestimmen Zustimmung oder Ablehnung bei der Auswahl der Wirksubstanzen.

Es ist auch möglich solche organischen Peroxide zu wählen, welche neben ihrer «peroxidisch»-bedingten Spontanwirkung in Form ihrer Zerfallsreste einen Konservierungsmitteleffekt auf den behandelten Materialien hinterlassen.

Geeignete Substanzen sind Acyl- und Diacylperoxide, Peroxidicarbonate, Alkyl- oder Diacylperverbindungen, Peracetale, Ketonperoxide, Persäuren und Hydroperoxide. Sie sind allein oder in Mischungen in wässrigen Aufbereitungen verwendbar.

Die reaktiven Eigenschaften der genannten Substanzen und Substanz-Gruppen können durch geeignete Aktivatoren modifiziert werden, so dass eine Steuerung von Reaktionszeit und Aktivierungstemperatur erreicht wird und damit auch eine geeignete Inaktivierungsphase eintreten kann.

Als Quelle für Mikrowellen können handelsübliche Mikrowellengeneratoren verwendet werden. Es bietet sich jedoch an, für das neue Verfahren Geräte zu entwickeln, die dem neuen Verwendungszweck optimal angepasst sind, da insbesondere die Dimensionen der handelsüblichen Geräte für das erfindungsgemässe Verfahren nicht optimal gewählt sind, so dass es zu unnötigem Totvolumen und nicht optimalem Ausnutzen der Mikrowellenenergie kommt.

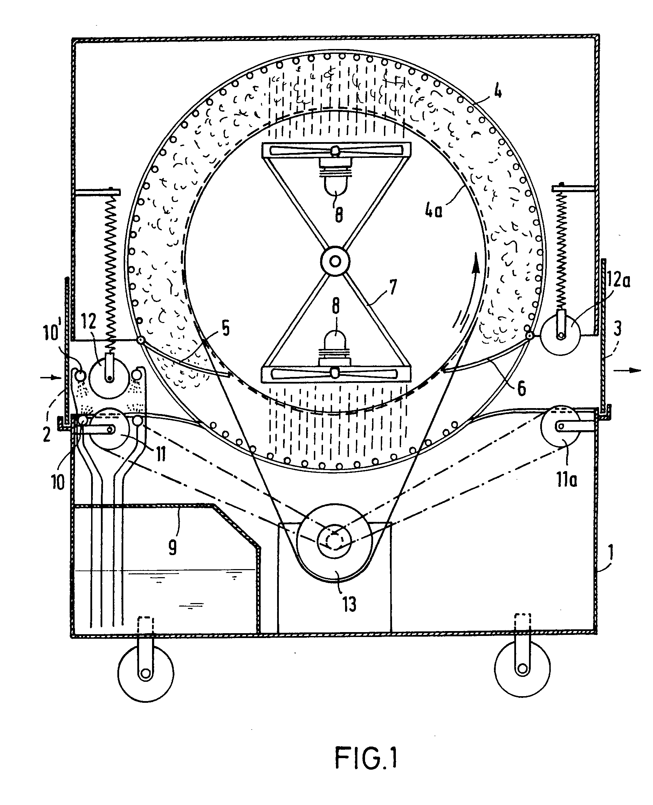

Die Ausführungsform der Vorrichtung zur Durchführung des Verfahrens zur chemothermischen Desinfektion und Sterilisation von Matratzen und grossvolumigen Bett-Textilien ist in Fig. 1 dargestellt und besteht aus einem vorzugsweise fahrbaren Behälter 1 mit einem verschliessbaren Einlassschlitz 2, einem verschliessbaren Auslassschlitz 3, einer Trommel 4, 4a einer Einlassklappe 5, einer Auslassklappe 6, einem zentral gelagertem, gegebenenfalls aktiv drehbaren Einsatz 7 mit mindestens einem Mikrowellensender 8, einem Flüssigkeitstank 9 und damit verbundenen Sprühdüsen 10 und 10'.

Um das Einführen und Herausholen des Materials zu erleichtern, sind hinter dem Einlassschlitz 2 eine angetriebene Rolle 11 und eine Andruckrolle 12 und gegebenenfalls vor dem Auslassschlitz 3 eine weitere angetriebene Rolle 11a und Andruckrolle 12a vorhanden.

Weiterhin ist die Trommel 4 als Rollenwand ausgebildet und schirmt die Mikrowellenstrahlung vor dem Austritt aus der Kammer ab. Die innere Trommelwand 4a ist aus einem mikrowellendurchlässigen Material, z.B. Kunststoff und wird durch den Motor 13 angetrieben, wodurch das Material aktiv transportiert wird.

Vor den Einlass- und Auslassschlitzen 2 und 3 können gewünschtenfalls aufklappbare Türen und Auflagetische angebracht werden, die die Handhabung erleichtern.

Die zu behandelnden Materialien wie Matratzen usw. werden in die Trommel 4, 4a eingefügt, wobei sie mittels der Sprühdüsen 10 und 10' befeuchtet werden. Danach schliesst sich der Einlassschlitz 2 und die Einlassklappe 5 und es schaltet sich der bzw. die Mikrowellensender 8 und der Motor 13 ein.

Nach Beendigung der Behandlung werden der Motor und der Sender abgeschaltet und der Auslassschlitz 3 sowie die Auslassklappe 6 geöffnet und das Material entnommen. Dadurch, dass Einlass und Auslass räumlich voneinander getrennt sind, kommt es im allgemeinen zu keiner Reinfektion des bereits behandelten Materials.

Als Mikrowellen kommen prinzipiell alle entsprechenden wärmeerzeugenden Wellen in Frage, jedoch werden in den meisten Ländern nur gewisse Frequenzen für die Wärmeerzeugung mit Mikrowellen zugelassen. In Ländern und Gegenden, wo derartige Beschränkungen nicht bestehen, können somit auch Geräte mit breitem Wellenspektrum zum Einsatz kommen.

Die überraschende Wirkung der erfindungsgemässen Vorrichtung ist in den nachfolgenden Beispielen näher beschrieben, wobei aus den Vergleichsversuchen die überraschend gute Wirksamkeit hervorgeht.

*Beispiel 1*

Als Testobjekte für die Sterilisation von Metallteilen beispielsweise den Federkernen in Matratzen usw. wurden Edelstahlschrauben von 25 mm Länge und 5 mm Durchmesser Feingewinde auf 0,1 ml suspendierter Gartenerde beimpft. Die Gartenerde enthielt u.a. ca. 10$^3$ bis 10$^4$ Klebsiella spec.-Keime und ca. 10$^6$ bis 10$^7$ Bac. subt. spec. Sporen. Die Schrauben wurden auf Filterpapier gelegt und mit Filterpapier abgedeckt. Unmittelbar vor der Mikrowellenbehandlung wurde 0,1 ml Wirkstofflösung auf das Filterpapier aufgetropft. Die Wirkstofflösung bestand aus wässrigem Alkohol in Konzentration von 0 bis 30%. Die Einwirkungszeit der Mikrowellen in Sekunden wurde von 30 bis 150 Sekunden variiert, wobei ein handelsübliches Mikrowellengerät mit 1100 W Ausgangsleistung (Privileg, Hitachi, Versandhaus Quelle) zur Anwendung kam. Die Schrauben wurden anschliessend auf Agarplatten ausgerollt und auf Keimwachstum untersucht. Die Auswertung der Ergebnisse erfolgte nach folgendem Schlüssel:

xxxx : massives Wachstum, Rasen  
xxx : lockerer Rasen  
xx : ca. 50 Kolonien unter der Auflage  
x : ca. 10 Kolonien  
O : keine Kolonienbildung

Die Ergebnisse sind in der nachfolgenden Tabelle 1 zusammengestellt:

Tabelle 1

| Zeit in Sek. \ Alkohol % | 0 | 5 | 10 | 20 | 30 |
|---|---|---|---|---|---|
| 30 | xxxx | xxxx | xxxx | xxxx | xxxx |
| Pause 15 + 60 | xxxx | xxxx | xxxx | xxx | xxx |
| Pause 15 + 90 | xxxx | xxxx | xxxx | xxx | xx |
| Pause 15 +120 | xxxx | xxx | xxx | xx | x |
| Kontrolle = 60% Alkohol xxxx | | | | | |

Zur Kontrolle wurden Schrauben mit 60%igem Alkohol 1 Std. ohne Mikrowellen-Einwirkung behandelt. Hierbei zeigten sich noch vereinzelte Bildung Gramnegativer Bakterienkolonien und massenhaft Bazillus-substilis-Kulturen.

Die Tabelle zeigt deutlich, dass weder der Alkohol, noch die Mikrowellen allein oder in Kombination miteinander in der Lage sind, die Desinfektion und Sterilisation zu erreichen.

*Beispiel 2*

Leinenläppchen wurden kontaminiert mit
a) Sporenerde ($10^6$ Sporen pro g)
b) einer Kotsuspension mit E. Coli, Enterokokken, Prot. vulg., Pseud. aerug., Staphylokokken, Candidahefen usw. $10^7$ - $10^8$ Keimen und $10^4$ Sporen des Bac. Subt. Typus/ml. Diese Lappen wurden jeweils 2 Min. einer Mikrowellenbehandlung gemäss Beispiel 1 ausgesetzt, wobei als Chemikalien $H_2O_2$, Formaldehyd, Glutardialdehyd und p-Chlor-m-Cresol eingesetzt wurden. Bestimmt wurde die Abtötungskonzentration der Wirkstofffe. Die Ergebnisse sind in der nachfolgenden Tabelle 2 zusammengestellt. Die Ergebnisse zeigen, dass in vergleichsweise sehr niedrigen Konzentrationen und sehr kurzen Einwirkungszeiten der Mikrowellen bereits hervorragende Ergebnisse erzielt werden.

Tabelle 2

| | Mischung vegetativer Keime [Kot-(1:4)]-Suspension* | Sporenerde Bac. subt. |
|---|---|---|
| $H_2O_2$ | 0,3 - 1,2% | 0,6% |
| Formaldehyd | 0,2 - 0,5% | 0,8 - 1,6% |
| Glutardialdehyd | 0,5% | 0,5 - 1,0% |
| P-Chlor-m-Kresol | 0,5 - 1,5% | >5% |

Bestimmung der biociden/sporiciden Konzentrationen bei 2minütiger Mikrowellen-Einwirkung

* ohne Berücksichtigung von Dauerformen.

*Beispiel 3*

In analoger Weise wie in Beispiel 2 beschrieben wurden Gummischlauchstücke als Keimträger mit Bac. subt. Sporen infiziert und einer Mikrowellenerhitzung ausgesetzt. Der Keimzahlnachweis in der Subkultur ergab, dass die Keimzahl von $10^6$ nach 30 Sekunden schon auf $10^3$, nach 60 Sekunden auf $10^2$ und nach 90 Sekunden auf 0 gesunken war, also Sterilität erzielt wurde.

*Beispiel 4*

Besonders problematisch ist die Desinfektion und Sterilisation von Matratzen. Um die Verhältnisse in Matratzen zu simulieren, wurden Keimträger (Leinenläppchen) zentral in vorbereiteten Taschen eines Matratzenschaumstoffblocks eingebracht. Der Schaumstoffblock hatte die Grösse von 20 × 20 × 10 cm und wurde vorher mit jeweils 120 g einer $H_2O_2$-Lösung getränkt. Diese Menge wurde durch Abpressen des überflüssigen Volumens eingestellt. Der keimtötende Effekt von stabilisierter $H_2O_2$-Lösung in Abhängigkeit von Konzentration und Zeit geht aus den folgenden Tabellen 3 und 4 hervor. Tabelle 3 zeigt die Ergebnisse mit der Kotsuspension gemäss Beispiel 2, Tabelle 4 die Ergebnisse mit Bac. subt. Sporen in Gartenerde. Die Keimzahleinsaat in Tabelle 3 betrug $10^8$ Keime, bei der Garten-erde 5 × $10^6$ Sporen. Die Kontrolle des Keimwachstums wurde auf Agarplatten mit Enthemmungsmitteln zur Neutralisation von Wirkstoffresten durchgeführt, und zwar nach 24, 48 und 72 Stunden bei 37°C. Die Auswertung des Keimwachstums erfolgte nach dem Schlüssel gemäss Beispiel 1. Die Temperatur des Schaumstoffblockes wurde durch Steuerung der Mikrowellen konstant zwischen 60 und 65°C gehalten.

Tabelle 3

| Konzentr. $H_2O_2$ in mg/ml \ Zeit Min. | 1 | 2 | 3 | 4 | 5 | Kontrolle |
|---|---|---|---|---|---|---|
| 3,5 | xxxx | xxx | xxx | xx | x | xxxx |
| 7,0 | xxxx | xxx | xx | x | 0 | xxxx |
| 10,5 | xxx | xx | x | 0 | 0 | xxxx |
| 14,0 | x | 0 | 0 | 0 | 0 | xxxx |
| 17,5 | 0 | 0 | 0 | 0 | 0 | xxxx |

Kurzzeitsterilisation von Kot (Keimträgerversuche)

### Tabelle 4

| Zeit Min. Konzentr. $H_2O_2$ in mg/ml | 1 | 2 | 3 | 4 | 5 | Kontrolle |
|---|---|---|---|---|---|---|
| 1,75 | xxx | xxx | x | x | 0 | xxxx |
| 3,5 | x | 0 | 0 | 0 | 0 | xxxx |
| 7,0 | 0 | 0 | 0 | 0 | 0 | xxxx |
| 10,5 | 0 | 0 | 0 | 0 | 0 | xxxx |
| 14,0 | 0 | 0 | 0 | 0 | 0 | xxxx |

Kurzzeitsterilisation von Sporenerde (Keimträgerversuche)

*Beispiel 5*

Für den Versuch wurde ein der Grösse nach passendes Modell einer Federkernmatratze angefertigt. Seine Masse waren 30 × 30 × 15 cm.

Die Polsterlagen bestanden aus

1. Bezug (schwerer Matratzendrell)
2. Bezug unter 1. (leichte Drellware)
3. Polsterwatte, gesteppt (8 bis 10 mm stark)
4. Seegrasfüllung (10 bis 15 mm stark)
5. Sackleinen worauf die Seegrasfüllung geheftet war
6. Ein Metallrahmengestell von 25 × 25 × 12 cm

Ausmass anstelle des Originalfederkerns zur Aufnahme der Edelstahlschrauben (s. Beispiel 1). Die Edelstahlschrauben dienen als schwer desinfizierbare Keimträger.

Als textile Keimträger wurden Leinenläppchen (vergleiche Beispiel 2 und 4) zwischen die einzelnen Polsterlagen plaziert, so dass für die Auswertung drei gleichzeitig entnommene Keimträgerproben zur Verfügung standen. Vor Plazierung der kontaminierten Keimträger wurde das Modell «Federkernmatratze» mit Hilfe einer Zweistoffdüsenleiste [Luft (3,0 bis 3,5 bar), Wirkstofflösung (drucklos)] ausserhalb der Behandlungskammer gleichmässig durchfeuchtet. Die Zusammensetzung der Wirkstofflösung war eine Kombination aus $H_2O_2$, Dibenzoylperoxid, Methyläthylketonperoxid in einer 10%igen Isopropanollösung mit einem Aktivsauerstoffgehalt von 12 mg/ml. Die Feuchtigkeitsaufnahme der Textilien- und Polsterlagen betrug 40 ± 5 ml. Das Gewicht der Polsterung betrug 420 g, das Gesamtvolumen des Modells 13,5 ℓ.

Die Mikrowellenbehandlung wurde in Intervallen der Mikrowellenbestrahlung und Pausen von je 30 Sekunden mit einer Gesamtzeit von 1 × = 1 Minute bis 8 × = 8 Minuten durchgeführt. Drei Keimträger wurden parallel entnommen und gelten als eine Probe. Die Auswertung erfolgte durch Rekultivierung der Restkeime auf

a) Agarplatten und
b) durch Kultur in Nährlösung.

Ohne Wertung der Restkeimdichte wurde Keimwachstum durch « + » und fehlendes Wachstum (steriler Befund) durch «O» in der Tabelle erfasst.

Die Ergebnisse sind in der nachstehenden Tabelle 5 zusammengestellt.

### Tabelle 5

| Behandlungsinterval | | Versuch mit Kotsuspension | | Versuche mit Sporenerde | | |
|---|---|---|---|---|---|---|
| Anzahl und Gesamtdauer | Behandlungsdauer | Agarkultur | Nährlösungskultur | Agarkultur | Nährlösungskultur | Bemerkungen |
| 0 | 0 | +/+/+ | +/+/+ | +/+/+ | +/+/+ | Kontrolle n. 48 Std. Bebrütungszeit bei 37°C |
| | M.-W. Gesamt in Sekunden | | | | | |
| 1 x = 1 Min. | 30  60 | +/0/+ | +/+/+ | +/+/0 | +/0/+ | n. 48 bis 72 Std. |
| 2 | 60  120 | +/0/0 | +/+/0 | 0/0/0 | +/+/0 | n. 72 Std. |
| 4 | 120  240 | 0/0/0 | 0/0/0 | 0/0/0 | 0/0/0 | n. 7 Tagen |
| 8 | 240  480 | 0/0/0 | 0/0/0 | 0/0/0 | 0/0/0 | n. 7 Tagen |

**Patentanspruch**

Vorrichtung zur chemothermischen Sterilisation von Matratzen und grossvolumigen Bett-Textilien mittels Wärme, die durch Mikrowellen erzeugt wird, sowie Zusatz von wässrigen oder wässrigalkoholischen Lösungen von Desinfektions- und Sterilisationsmitteln, bestehend aus einem vorzugsweise fahrbaren Behälter (1) mit einem verschliessbaren Einlassschlitz (2), einem verschliessbaren Auslassschlitz (3), einer Trommel (4), (4a), einer Einlassklappe (5), einer Auslassklappe (6), einem zentral gelagerten, gegebenenfalls aktiv drehbaren Einsatz (7) mit mindestens einem Mikrowellensender (8), einem Flüssigkeitstank (9) und damit verbundenen Sprühdüsen (10, 10'), ferner aus einer, hinter dem Einlassschlitz (2) angeordneten, angetriebenen Rolle (11) und einer Andruckrolle (12) und gegebenenfalls einer, vor dem Auslassschlitz (3) vorhandenen, weiteren angetriebenen Rolle (11a) und Andruckrolle (12a), wobei die Trommelwand (4) als Rollenwand ausgebildet ist und die innere Trommelwand (4a) über einen Motor (13) angetrieben wird.

## Claim

A device for the chemothermal sterilization of mattresses and large-volume bed textile materials by means of heat generated by microwaves and addition of aqueous or aqueous-alcoholic solutions of disinfectants and sterilizing agents, consisting of a preferably movable container (1) with a sealable inlet slot (2), a sealable outlet slot (3), a drum (4), (4a), an inlet flap (5), an outlet flap (6), a centrally supported inset (7), which optionally is actively rotatable, with at least one microwave emitter (8), a liquid storage tank (9) and spray nozzles (10, 10') connected therewith, further a driven roll (11) located behind the inlet slot (2) and a pressing-contact roll (12) and optionally a further driven roll (11a) located in front of the outlet slot (3) and pressing-contact roll (12a), the drum wall (4) being shaped in the form of a roll wall and the inner drum wall (4a) being driven by a motor (13).

## Revendication

Dispositif pour la stérilisation thermochimique de matelas et de literies de grand volume à l'aide de la chaleur produite par des micro-ondes, ainsi que de l'addition de solutions aqueuses ou hydroalcooliques de produits de désinfection et de stérilisation, dispositif consistant en un récipient (1) de préférence mobile comportant une fente d'admission (2) obturable, une fente d'évacuation (3) obturable, un tambour (4), (4a), un clapet d'admission (5), un clapet d'évacuation (6), un organe central (7), éventuellement tournant pendant la marche, comportant au moins un émetteur (8) de micro-ondes, un réservoir (9) de liquide auquel sont reliées des buses (10, 10'); ainsi qu'en un rouleau mené (11) et un rouleau presseur (12) disposés derrière la fente d'admission (2) et éventuellement un autre rouleau mené (11a) et un autre rouleau presseur (12a) disposés devant la fente d'évacuation (3), la paroi (4) du tambour ayant la forme d'une paroi circulaire et la paroi interne (4a) du tambour étant entraînée par un moteur (13).

FIG.1